# EUROPÄISCHE PATENTSCHRIFT

(11) **EP 2 420 285 B1**
(45) Veröffentlichungstag und Bekanntmachung des Hinweises auf die Patenterteilung: **03.06.2020**
(21) Anmeldenummer: 11177981.5
(22) Anmeldetag: 18.08.2011
(51) Int. Cl.: A61M 39/12, A61B 18/02, F16L 33/00, F16L 37/092, A61M 39/10, A61B 17/3203

(54) **Griffelement oder einen Stecker eines Chirurgieinstruments mit einer Vorrichtung zum dichtenden Anschluss eines Druckschlauches**
Grip element or connector of a surgical instrument comprising a device for sealing connection of a pressure hose
Poignée ou élément de raccordement d'un instrument chirurgical avec un dispositif de raccordement étanche d'un tuyau flexible

(30) Priorität: 18.08.2010 DE 102010037027
(43) Veröffentlichungstag der Anmeldung: 22.02.2012
(73) Patentinhaber: Erbe Elektromedizin GmbH, 72072 Tübingen (DE)
(72) Erfinder: Sick, Christian, 72141 Nehren (DE); Amann, Markus, 72070 Tübingen (DE)
(74) Vertreter: Rüger Abel Patentanwälte PartGmbB

(56) Entgegenhaltungen:
- FR-A1- 2 562 984
- US-A- 4 431 216
- US-A- 4 712 813
- US-A- 5 174 611
- US-A1- 2003 028 182

## Beschreibung

Die Erfindung beruht auf einer speziellen Vorrichtung zum dichtenden Anschluss eines Druckschlauches an ein Griffelement oder einen Stecker eines Chirurgieinstrumentes, umfassend wenigstens ein Dichtelement, das in einem Montagezustand in einem Anschlussbereich dichtend zwischen dem Griffelement und dem Druckschlauch angeordnet ist.

Die Erfindung betrifft demzufolge insbesondere einen Handgriff für ein Chirurgieinstrument, insbesondere ein Kryochirurgieinstrument mit einem Griffelement zum Halten des Chirurgieinstruments und wenigstens einem Druckschlauch, dessen Griffelement über eine Vorrichtung gemäß der vorgenannten Art angeschlossen oder anschließbar ist.
Derartige Anschlussvorrichtungen und Handgriffe, die über derartige Anschlussvorrichtungen verfügen, sind aus dem Stand der Technik bekannt. Ihre Anwendung finden sie in der Geräte-gestützten Chirurgie und insbesondere in der Kryochirurgie oder Wasserstrahlchirurgie, bei der über ein entsprechend ausgerüstetes Chirurgieinstrument Operationen am menschlichen oder tierischen Gewebe möglich sind. Meist ist es bei solchen Chirurgieinstrumenten nötig, über einen Druckschlauch ein Applikationsfluid oder dergleichen Wirkfluid einem Griffelement zuzuführen, von wo es dann einem Applikationskopf, beispielsweise einer Applikationsdüse oder einer Kryosonde zugeleitet wird.

Aufgrund der Sterilitätsanforderungen, die für solche Chirurgieinstrumente gelten, werden an die Anschlussvorrichtung zum Anschluss des oder der Druckschläuche am Griffelement hohe Anforderungen gestellt. Insbesondere bei solchen Griffinstrumenten, die nach dem Gebrauch einer maschinellen Reinigung, beispielsweise einer dafür geeigneten Waschmaschine zugeführt werden, besteht ein hoher Bedarf, die Anschlussvorrichtung so auszubilden, dass sie einen dauerhaft dichten und darüber hinaus preiswerten Anschluss zwischen Druckschlauch und Griffelement oder einem daran vorgesehenen Stecker garantiert.

Aus dem Stand der Technik sind Anschlussvorrichtungen bzw. Handgriffe für Chirurgieinstrumente bekannt, bei denen der Druckschlauch über eine Schraubverbindung mit dem Griffelement verbunden wird. Meist wird dazu ein O-Ring oder dergleichen Dichtungselement axial verpresst, wodurch eine radial dichtende Verbindung entsteht, die den Druckschlauch dichtend am Griffelement befestigt. Über die Verschraubung wird darüber hinaus der Druckschlauch so gequetscht, dass eine axiale auf den Schlauch einwirkende Zuglast abgetragen werden kann. Die Druckschriften US 5174611 A und US 4431216 A offenbaren darüber hinaus Kupplungen mit einem Schnellverbindungsmechanismus für Druckschläuche.

Als problematisch hat sich hier herausgestellt, dass über die geforderte Aufbereitungszahl eine lösungssichere Verbindung aufgrund des Setz- bzw. Fließverhaltens des Kunststoffdruckschlauches nicht gesichert werden kann. Darüber hinaus entstehen manchmal durch die axiale Pressung des O-Ringes aufgrund der hohen Anpresskräfte Verformungen, die über dem zulässigen Bereich liegen und so zum vorzeitigen Versagen bzw. zu einen irreversiblen Verformung des Dichtelementes führen.
Aufgabe der vorliegenden Erfindung ist es folglich, eine Vorrichtung zum dichtenden Anschluss eines Druckschlauches an ein Griffelement eines Chirurgieinstrumentes bzw. einen Handgriff für ein solches Chirurgieinstrument darzubieten, der einen dauerhaft dichten Anschluss des Druckschlauches an das Griffelement garantiert.
Insbesondere ist es nötig, dass die Verbindung zwischen Druckschlauch und Griffelement einem Arbeitsdruck zwischen etwa 40 bar und 65 bar (Sicherheitsgrenze 130 bar) standhalten und zudem absolut fluid- und insbesondere gasdicht sein muss. Ebenso müssen die verwendeten Dichtelemente einer mindestens 100maligen Aufbereitung bei 138°, nämlich einem Waschen und Sterilisieren standhalten.

Es sei darauf hingewiesen, dass hiermit auch der Anschluss eines Druckschlauches an einen Stecker gemeint ist, der dann als Kopplungselement zu einer Fluidquelle oder einem Verbraucher in einer chirurgischen Anwendung dient.

Obige Aufgabe wird durch die Kombination der Merkmale gemäß Patentanspruch 1 gelöst.

Insbesondere umfassen die erfindungsgemäßen Gegenstände eine Vorrichtung zum dichtenden Anschluss eines Druckschlauches an ein Griffelement eines Chirurgieinstrumentes oder an einen Stecker gelöst, umfassend wenigstens ein Dichtelement, das in einem Montagezustand in einem Anschlussbereich dichtend zwischen dem Griffelement (oder Stecker) und dem Druckschlauch angeordnet oder anordbar ist, wobei ein Stützelement, das im Anschlussbereich im Innenraum des Druckschlauches derart angeordnet ist, dass das Dichtelement zwischen dem Stützelement und dem Druckschlauch auf der einen Seite und dem Griffelement auf der anderen Seite eingeklemmt oder einklemmbar ist.

Darüber hinaus wird diese Aufgabe durch einen Handgriff für ein Chirurgieinstrument, insbesondere Kryochirurgieinstrument gelöst, mit einem Griffelement zum Halten des Chirurgieinstruments, und wenigstens einem Druckschlauch, der am Griffelement über die oben beschriebene Vorrichtung angeschlossen oder anschließbar ist.

Ein wesentlicher Punkt der Erfindung ist also die Anordnung eines Stützelementes im Anschlussbereich derart, dass es von der Innenseite des Druckschlauches aus so auf das Dichtelement wirkt, dass das Dichtelement zwischen dem Stützelement und dem Druckschlauch auf der einen Seite und dem Griffelement auf der anderen Seite eingeklemmt ist oder einklemmbar ist. Bei einer solchen Ausführungsform ist das Dichtelement vorzugsweise senkrecht zur Axialrichtung des Druckschlauches im Anschlussbereich belastet und vorzugsweise verformt, wobei diese Belastungsrichtung bei der erfindungsgemäßen Vorrichtung entscheidend zur dauerhaften Dichtwirkung auch bei den zuvor genannten thermischen Belastungen beiträgt; das Dichtelement wird vorzugsweise radial gepresst.
Bei der radial zur Achse des Druckschlauches verlaufenden Belastung des Dichtelementes verhindert das Stützelement entscheidend eine Verformung des Druckschlauches im Angriffspunkt des Dichtelementes, so dass die Dichtwirkung entscheidend verbessert wird.

Das Dichtelement ist dabei vorzugsweise in einer Dichtelementaufnahme am Griffelement gelagert und insbesondere so, dass die Kombination aus Stützelement und Druckschlauch so in das Griffelement eingeschoben werden kann, dass das Dichtelement dichtend daran anliegt, wodurch der Übergangsbereich zwischen Druckschlauch und Griffelement dicht versiegelt wird.

Natürlich ist es auch möglich, das Dichtelement schon vor dem Einsetzen des Druckschlauches in das Griffelement mit dem Druckschlauch in Wirkverbindung zu bringen und dann die Anordnung aus Dichtelement, Stützelement und Druckschlauch dichtend in das Griffelement einzuführen.

Vorzugsweise weist das Dichtelement wenigstens einen O-Ring oder ein dergleichen den Druckschlauch und das Stützelement im Montagezustand umlaufendes Hüllelement auf, wobei der Montagezustand der Zustand ist, in der der Druckschlauch dichtend mit dem Griffelement verbunden ist.

Vorzugsweise weist das Dichtelement also einen O-Ring oder ein dergleichen den Druckschlauch und das Stützelement umlaufendes Hüllelement auf, das im Montagezustand so auf den Druckschlauch aufgeschoben ist bzw. auf den Druckschlauch aufschiebbar ist, dass es dichtend an diesem anliegt und im Zusammenwirken mit dem Griffelement und insbesondere mit einer Innenwandung eines Aufnahmebereichs des Griffelements einen dichtenden Übergang zwischen dem Druckschlauch und dem Griffelement bildet.

Auch hier ist es also möglich, den O-Ring bzw. das Hüllelement, wobei im Folgenden, wenn vom O-Ring die Rede Ist, auch immer ein Hüllelement gemeint sein kann, im Griffelement und insbesondere einer Dichtelementaufnahme am Griffelement anzuordnen und anschließend den Druckschlauch zusammen mit dem Stützelement in den O-Ring so einzuführen, dass ein dichtender Anschluss entsteht, oder aber den O-Ring bereits über das Stützelement und den Druckschlauch überzustülpen und anschlie-ßend die Kombination aus Stützelement, Druckschlauch und O-Ring in das Griffelement und insbesondere einen Aufnahmebereich, der eine geeignete Aussparung, beispielsweise die Dichtelementaufnahme aufweist, einzuführen.

Vorzugsweise weist das Dichtelement wenigstens zwei O-Ringe oder dergleichen den Druckschlauch und das Stützelement umlaufende Hüllelemente auf, die über wenigstens ein Distanzelement voneinander entkoppelt sind. Auch hier ist, wenn von den O-Ringen die Rede ist, automatisch auch das Hüllelement mit umfasst. Die Entkopplung der beiden O-Ringe über das Distanzelement stellt eine zusätzliche Verbesserung der Dichtwirkung dar.
Vorzugsweise ist das Distanzelement als eigenständiges Bauteil in einer Distanzelementaufnahme am Griffelement gelagert oder lagerbar, insbesondere in axialer Richtung des Hüllschlauches im Anschlussbereich zwischen den beiden O-Ringen oder den dergleichen Hüllelementen. Wie schon zuvor unter Bezug auf die O-Ringe erwähnt, ist es auch hier möglich, das Distanzelement vor dem Einsetzen des Druckschlauches in das Griffelement in eine Distanzelementaufnahme am Griffelement einzusetzen und insbesondere mit den beiden O-Ringen auszurichten oder aber das Distanzelement und wenigstens einen O-Ring vor dem Einsetzen auf den Druckschlauch aufzuschieben.
Das Distanzelement ist vorzugsweise als Ring bzw. Hülse ausgebildet und auf den Druckschlauch aufschiebbar. Die Abmessungen des Distanzelementes und insbesondere eines als Distanzring oder -hülse ausgebildeten Distanzelementes sind dabei derart gewählt, dass sie in Bezug auf den Außendurchmesser geringfügig kleiner sind als das oder die Dichtelemente, so dass sich die Dichtelemente unter Optimierung der Dichtwirkung verformen können, ohne dass diese Verformung vom Distanzelement behindert wird.

Erfindungsgemäß ist das Stützelement als eine Stützhülse ausgebildet, die insbesondere einen im Vergleich zum Innenraumdurchmesser des Hüllschlauches komplementären und insbesondere derart geringfügig größeren Außendurchmesser aufweist, dass das Stützelement im Innenraum des Hüllschlauches diesen aufdehnt. Das Stützelement ist auf diese Weise fest im Innenraum des Druckschlauches angeordnet, so dass insbesondere das Einführen des Druckschlauches in einen Aufnahmebereich des Griffelementes und insbesondere das Einstülpen in die O-Ringe bzw. Hüllelemente sicher möglich ist.
Vorzugsweise sind also die O-Ringe mit ihrer Rotationsachse so angeordnet, dass diese parallel zur Achse des Druckschlauches und parallel zur Achse eines möglicherweise vorhandenen Distanzelementes angeordnet sind. Auf diese Weise können das Stützelement und der Druckschlauch axial In die O-Ringe bzw. das Hüllelement eingeschoben werden, so dass es zu einem dichtenden Anschluss an das Griffelement im Montagezustand kommt.

Erfindungsgemäß ist am Druckschlauch wenigstens ein Halteelement angeordnet, das mit einem Anschlagelement am Griffelement zur Fixierung des Druckschlauches insbesondere in axialer Richtung des Druckschlauches im Anschlussbereich in Wirkverbindung steht. Eine solche Anordnung garantiert die sichere Fixierung des Druckschlauches im Griffelement.

Weiterhin erfindungsgemäß weist das Halteelement einen Crimpring auf, der ortsfest auf der Außenseite des Druckschlauches über der Stützhülle verpresst ist. Ein solcher Crimpring oder ein dergleichen Fixierelement kann also beispielsweise auf die Außenseite des Druckschlauches aufgeschoben und dort verpresst oder sonstwie fixiert werden. Hier sind sämtliche aus dem Stand der Technik bekannte Verfahren zur Anordnung eines Halteelementes auf einem Druckschlauch anwendbar, auch wenn diese eventuell nicht gemäß der vorliegenden Erfindung sind.

Vorzugsweise weist der Crimpring oder das dergleichen Fixierelement wenigstens einen Fixierfortsatz auf, der im angeordneten Zustand auf der Außenseite des Druckschlauches in Richtung des Druckschlauchinneren vorsteht. Unter angeordnetem Zustand wird im Umfang der Erfindung natürlich der Zustand verstanden, bei dem der Crimpring oder dergleichen Fixierelement auf dem Druckschlauch angeordnet sind. Die Ausbildung eines Fixierfortsatzes gemäß der vorgenannten Art verbessert die Kraftabtragung des Crimpringes oder des dergleichen Fixierelementes, so dass auch sehr hohe Kräfte und insbesondere axial auf den Druckschlauch wirkende Kräfte vom Halteelement abgetragen werden können.

Vorzugsweise weist das Anschlagelement wenigstens ein Befestigungsmittel auf, über das es mit dem Griffelement und/oder mit dem Halteelement in einem insbesondere lösbaren Arretierungs- oder Stützeingriff bringbar ist. Das Anschlagelement kann also beispielsweise ein Rastring sein, der über den Druckschlauch übergeschoben bzw. überschiebbar ist und nach dem Einsetzen des Druckschlauches in Kombination mit dem darauf angeordneten Halteelement mit dem Griffelement so verrastbar ist, dass es das Halteelement fixiert und insbesondere bei einer axialen Zugbelastung auf den Druckschlauch gegen ein Herausziehen aus dem Griffelement abstützt. Das Anschlagelement kann dabei also beispielsweise als Rastring vorgesehen sein, der mit dem Griffelement verrastbar ist. Natürlich ist es auch möglich, das Anschlagelement als Bajonettverschlusselement auszuführen, das über einen Bajonettverschluss mit dem Griffelement arretiert ist und so Kräfte, die über das Halteelement eingeleitet werden, in das Griffelement ableitet. Hier sind sämtliche aus dem Stand der Technik bekannte Vorrichtungen zur Fixierung des Anschlagelementes gegen das Griffelement bzw. zur Arretierung oder Abstützung des Halteelementes am Anschlagelement anwendbar.

Vorzugsweise ist am freien distalen Ende des Druckschlauches und/oder des Stützelementes, also an dem Ende, mit dem der Druckschlauch bzw. das Stützelement in das Griffelement und insbesondere in die O-Ringe oder dergleichen Hüllelemente eingeführt werden, ein Einführelement angeordnet, das derart ausgebildet ist, dass es das Einführen des Druckschlauches zusammen mit dem Stützelement in den Aufnahmebereich des Griffelementes und insbesondere in den Innenraum des O-Rings oder des dergleichen Hüllelementes bzw. eines eventuell vorgesehenen Distanzelementes unterstützt. Ein solches Einführelement kann beispielsweise ein entsprechend angeschrägt ausgeführtes distales Ende des Druckschlauches und/oder des Stützelementes sein, so dass die daraus resultierenden Führungsflächen das Einführen des Druckschlauches zusammen mit dem Stützelement erleichtern.

Wie bereits zuvor erwähnt, betrifft die Erfindung auch einen Handgriff für ein Chirurgieinstrument, insbesondere Kryochirurgieinstrument, mit einem Griffelement zum Halten des Chirurgieinstruments, und wenigstens einem Druckschlauch, der an ein Griffelement über eine Vorrichtung gemäß der zuvor genannten Arten angeschlossen oder anschließbar ist. Aus Redundanzgründen wird auf erneute Erläuterungen zur Ausführung dieser Vorrichtung verzichtet und explizit auf die vorhergehenden Passagen verwiesen.

Weitere Ausführungsformen der Erfindung ergeben sich aus den Unteransprüchen.

Im Folgenden wird die Erfindung anhand eines Ausführungsbeispiels beschrieben, das durch die beiliegenden Zeichnungen näher erläutert wird. Hierbei zeigen:
- Fig. 1: eine schematisch Darstellung einer Ausführungsform eines Chirurgieinstrumentes;
- Fig. 2: eine Detailansicht eines Anschlussbereiches eines Druckschlauches an einem Griffelement des Chirurgieinstrumentes gemäß Fig. 1; und
- Fig. 3: einen Querschnitt durch den Anschlussbereich aus Fig. 2 gemäß der Schnittführung aus Fig. 2.

Im Folgenden werden für gleiche und gleich wirkende Bauteile dieselben Bezugsziffern verwendet, wobei zur Unterscheidung bisweilen Hochindizes ihre Anwendung finden.

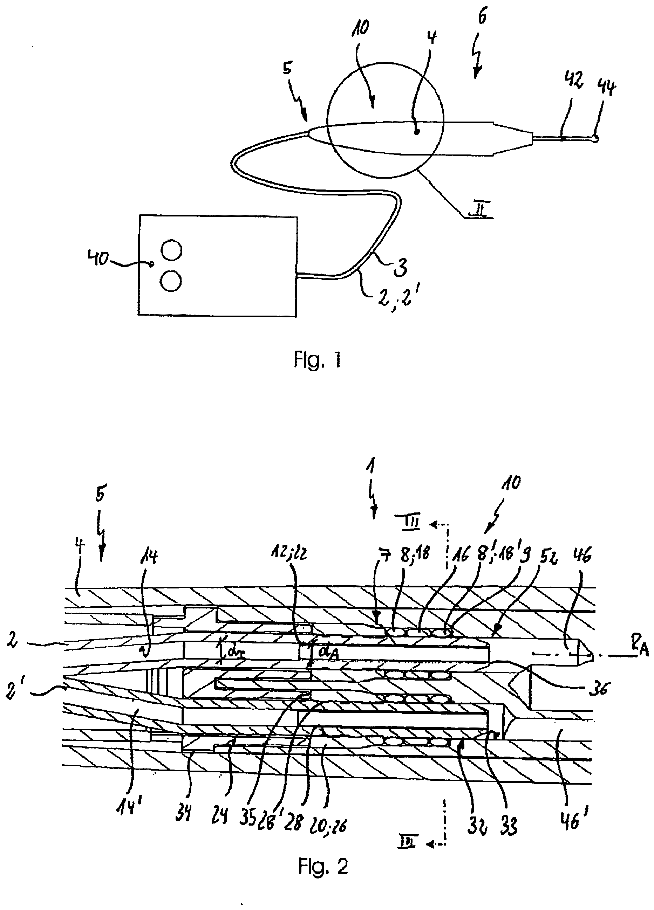

Fig. 1 zeigt eine Ausführungsform eines Chirurgieinstrumentes 30, wie es beispielsweise bei der Kryochirurgie oder der Wasserstrahlchirurgie seine Anwendung findet.

Dargestellt ist eine Versorgungseinheit 40, die einen Handgriff 6 über einen Schlauchsatz 3 mit einem Applikationsfluid versorgt. Der Schlauchsatz 3 umfasst dabei zwei Druckschläuche 2, von denen einer als Zuleitungsschlauch, der andere als Ableitungsschlauch verwendet wird, um das Applikationsfluid von der Versorgungseinheit 40 zum Handgriff 6 zu- bzw. abzuleiten.

Der Handgriff 6 seinerseits umfasst ein Griffelement 4, das der Aufnahme des Schlauchsatzes 3 bzw. der Druckschläuche 2 und einer Applikationssonde 42 dient, die beispielsweise als Kryochirurgiesonde mit einer koaxialen Leitungsführung ausgebildet sein kann. Das bedeutet, dass das über die Versorgungseinheit 40 zugeführte Applikationsfluid über einen Druckschlauch 2 in das Griffelement 4 geführt wird und von dort über eine entsprechende Druckschlauchführung in die Applikationssonde 42 geleitet wird. Dort wird das Applikationsfluid in einer äußeren koaxialen Leitungsführung, also beispielsweise auf der Außenseite der Applikationssonde 42 zum Applikationssondenkopf 44 geleitet und in einer inneren koaxialen Leitungsführung zurückgeführt, um im Griffelement 6 in den zweiten rückführenden Druckschlauch 2' eingeleitet zu werden, um zur Versorgungseinheit 40 zurückzufließen.

Bei dem hier dargestellten Chirurgieinstrument 30 und insbesondere bei einem standardisierten Kryochirurgieinstrument beträgt der Arbeitsdruck des Applikationsfluides im Druckschlauch 2 und insbesondere in dem in Fig. 2 dargestellten und im Folgenden noch detailliert erläuterten Anschlussbereich 10 zwischen 40 und 65 bar, wobei eine Sicherheitsgrenze von 130 bar gegeben sein soll. Im chirurgischen Arbeitsumfeld ist dabei 100%ige Fluid- und Gasdichtigkeit Voraussetzung. Insofern sind an den Anschlussbereich 10 (siehe auch Flg. 2) zwischen dem Schlauchsatz 3 bzw. den Druckschläuchen 2 und dem Griffelement 4 des Handgriffes 6 besondere Anforderungen zu stellen.

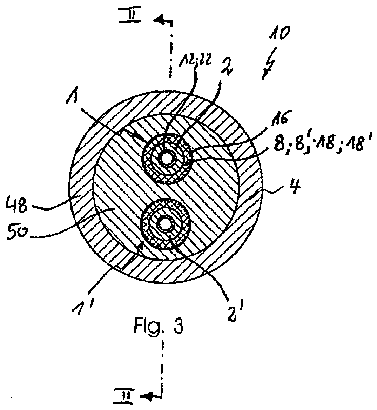

Um hier den sicheren Anschluss der Druckschläuche 2 an das Griffelement 4 des Handgriffes 6 zu gewährleisten, weist die erfindungsgemäße Anschlussvorrichtung 1 bzw. der erfindungsgemäße Handgriff 6 folgenden Aufbau auf, der insbesondere in den Fig. 2 und 3 dargestellt ist:
So zeigt Fig. 2 einen Längsschnitt durch den Anschlussbereich 10 zwischen Druckschlauch 2 und Griffelement 4 gemäß der Schnittführung aus Fig. 3 und Fig. 3 einen Querschnitt durch den Anschlussbereich 10 gemäß der Schnittführung aus Fig. 2.

Erkennbar ist das Griffelement 4, in das von seinem proximalen Ende 5 aus (siehe auch Fig. 1) die beiden Druckschläuche 2 eingeführt und dichtend daran befestigt sind. Die Druckschläuche 2 sind dabei so angeordnet, dass sie mit weiterführenden Leitungselementen 46 in Fluidverbindung stehen, um die von der Versorgungseinheit 40 (siehe Fig. 1) zugeführten Applikationsfluide der Applikationssonde 42 zuzuführen.

Ein jeder Druckschlauch 2 ist über eine Ausführungsform der erfindungsgemäßen Anschlussvorrichtung 1 am Griffelement 4 befestigt.

Die Anschlussvorrichtung 1 umfasst dabei zwei Dichtelemente 8, die in Form von O-Ringen 18 in einer Dichtelementaufnahme 9 angeordnet sind. Die Dichtelementaufnahme 9 ist ein Teil eines Aufnahmebereiches 7, der so im Griffelement 4 ausgebildet ist, dass er das Einführen des Druckschlauches 2 und weiterer, im Folgenden noch detailliert beschriebener Komponenten erlaubt.

In Axialrichtung R_{A} des (montierten) Druckschlauches 2 ist zwischen den beiden Dichtelementen 8 bzw. O-Ringen 18 ein als eigenständiges Bauteil ausgeführtes Distanzelement 16 angeordnet, das hier als Distanzelementring ausgeführt ist und die beiden Dichtelemente 8 bzw. O-Ringe 18 voneinander entkoppelt.

In Fig. 3 Ist dieses Distanzelement 16 gestrichelt dargestellt. Wie ebenfalls in Fig. 3 dargestellt, besteht das Griffelement 4 hier aus einer äußeren Griffelementhülle 48 und einem inneren Griffelementkern 50, in dem die Anschlussvorrichtung 1 ausgebildet ist und der darüber hinaus die weiterführenden Leitungselemente 46 aufweist.

Erfindungsgemäß weist jeder Druckschlauch 2 in seinem Innenraum 14 ein als Stützhülse 22 ausgebildetes Stützelement 12 auf, das so in den Innenraum 14 des Druckschlauches 2 eingeschoben ist, dass es passgenau dort fixiert ist und insbesondere den Druckschlauch 2 geringfügig aufweitet. Die Stützhülse 22 ist insbesondere so ausgebildet, dass sie einen zum Innenraumdurchmesser d₁ des Druckschlauches 2 komplementären und insbesondere derart geringfügig größeren Außendurchmesser d_{A} aufweist, dass das Stützelement 12 im Innenraum 14 des Druckschlauches 2 diesen aufdehnt.

Am freien distalen Ende 52 des Druckschlauches 2 und des Stützelementes 12 ist bei dieser Ausführungsform ein Einführelement 36 ausgebildet, das das einfache Einführen des Druckschlauches 2 und des Stützelementes 12 in den Aufnahmebereich 7 des Griffelementes 4 bzw. des inneren Kerns 50 erlaubt. Bei dieser Ausführungsform ist das Einführelement 36 durch abgeschrägte Eckbereiche des freien distalen Endes 52 des Druckschlauches 2 gebildet.

Zum dichtenden Anschluss des Druckschlauches 2 an das Griffelement 4 sind die als O-Ringe 18 ausgeführten Dichtelemente 8 über den Druckschlauch 2 gestülpt und derart ausgebildet, dass sie nach dem Einführen des Druckschlauches 2 in den Aufnahmebereich 7 des Griffelementes 4 eine dichtende Verbindung zwischen der Außenseite 32 des Druckschlauches 2 und der Innenseite 33 des Griffelementes 4 im Anschlussbereich 10 bzw. Aufnahmebereich 7 sicherstellen. Aufgrund der Anordnung der Dichtelemente 8 und deren radialer Pressung kommt es nur zu geringfügigen Verformungen, so dass auch bei höheren Temperatureinflüssen dauerhaft ein dichtender Anschluss gewährleistet bleibt.

Zur Lagesicherung des Druckschlauches 2 insbesondere in Axialrichtung R_{A} ist auf der Außenseite 32 jedes Druckschlauches 2 ein Halteelement 20 in Form eines Crimpringes 26 ausgebildet und insbesondere auf die Außenseite 32 aufgepresst oder dergleichen fixiert. Zur Verbesserung dieser Fixierung weist das Halteelement 20 Fixierfortsätze 28 auf, die in Richtung des Innenraumes 14 des Druckschlauches 2 vorstehen.

Dieses Halteelement 20 bzw. der Crimpring 26 stehen mit einem Anschlagelement 24 in Wirkverbindung, das über Befestigungsmittel 34 am Griffelement 4 so befestigt ist, dass es ein Herausziehen des Druckschlauches 2 in Axialrichtung R_{A} aus dem Aufnahmebereich 7 des Griffelementes 4 verhindert. Dazu steht das Anschlagelement 24 mit einem Stützfortsatz 35 derart am Halteelement 20 bzw. dem Crimpring 26 an, dass Zugkräfte, die vom Druckschlauch 2 in das Halteelement 20 eingetragen werden, über den Stützfortsatz 35 und das Befestigungsmittel 34 und von dort in das Griffelement 4 eingetragen werden.

Das Befestigungsmittel 34 kann beispielsweise ein Schraubverbindungsmittel, ein Rastverbindungsmittel etc. sein, wie es aus dem Stand der Technik bekannt ist, um ein Anschlagelement 24 mit einem Griffelement 4 zu verbinden.

Aus obigem ist ersichtlich, dass die Erfindung auch zur Verbindung zwischen einem Druckschlauch und einem Stecker dienen kann, der dann in die Versorgungseinheit (oder ein Griffelement) oder aber in eine Verlängerungskupplung gesteckt werden kann.

### Bezugszeichenliste

- 1: Anschlussvorrichtung
- 2: Druckschlauch
- 3: Schlauchsatz
- 4: Griffelement
- 5: Proximales Ende des Griffelementes
- 6: Handgriff
- 7: Aufnahmebereich
- 8: Dichtelement
- 9: Dichtelementaufnahme
- 10: Anschlussbereich
- 12: Stützelement
- 14: Innenraum des Druckschlauches
- 16: Distanzelement
- 18: O-Ring
- 20: Halteelement
- 22: Stützhülse
- 24: Anschlagelement
- 26: Crimpring
- 28: Fixierfortsatz
- 30: Chirurgieinstrument
- 32: Außenseite
- 33: Innenseite
- 34: Befestigungsmittel des Anschlagelementes
- 35: Stützfortsatz
- 36: Einführelement
- 38: Innenraum des O-Rings
- 40: Versorgungseinheit
- 42: Applikationssonde
- 44: Applikationssondenkopf
- 46: Leitungselement
- 48: Griffelementhülse
- 50: Innerer Kern
- d₁: Innendurchmesser
- d_{A}: Außendurchmesser
- R_{A}: Axialrichtung

## Patentansprüche

1. Stecker eines Chirurgieinstruments (30) oder Handgriff für ein Chirurgieinstrument (30) mit einem Griffelement (4) zum Halten des Chirurgieinstruments (30) und wenigstens einem Druckschlauch (2), der an dem Griffelement (4) oder dem Stecker über eine Vorrichtung (1) angeschlossen ist, mittels derer der Druckschlauch (2) dichtend an das Griffelement (4) oder den Stecker des Chirurgieinstrumentes (30) angeschlossen ist, umfassend wenigstens ein Dichtelement (8), das in einem Montagezustand in einem Anschlussbereich (10) dichtend zwischen dem Griffelement (4) und dem Druckschlauch (2) angeordnet ist,
mit einem Stützelement (12), das als Stützhülse (22) ausgebildet ist und im Anschlussbereich (10) im Innenraum (14) des Druckschlauches (2) derart angeordnet ist, dass das Dichtelement (8) zwischen dem Stützelement (12) und dem Druckschlauch (2) auf der einen Seite und dem Griffelement (4) auf der anderen Seite eingeklemmt ist,
wobei an dem Druckschlauch (2) wenigstens ein Halteelement (20) angeordnet ist, das einen Crimpring (26) aufweist, der ortsfest auf der Außenseite (32) des Druckschlauches (2) über der Stützhülse verpresst ist, wobei das Haltelement (20) mit einem Anschlagelement (24) am Griffelement (4) zur Fixierung des Druckschlauches (2) in axialer Richtung (R_{A}) des Druckschlauches (2) in Wirkverbindung steht, wobei der Crimpring (26) mit dem Anschlagelement im Anschlussbereich (10) in Wirkverbindung steht.

2. Vorrichtung nach Anspruch 1, **dadurch gekennzeichnet, dass** das Dichtelement (8) wenigstens einen O-Ring (18) oder ein dergleichen den Druckschlauch (2) und das Stützelement (12) im Montagezustand umlaufendes Hüllelement aufweist.

3. Vorrichtung nach einem der vorhergehenden Ansprüche, **dadurch gekennzeichnet, dass** das Dichtelement (8) wenigstens zwei O-Ringe (18) oder dergleichen den Druckschlauch (2) und das Stützelement (12) umlaufende Hüllelemente aufweist, die über wenigstens ein Distanzelement (16) von einander entkoppelt sind.

4. Vorrichtung nach einem der vorhergehenden Ansprüche, **dadurch gekennzeichnet, dass** das Stützelement (12) eine Stützhülse (22) aufweist, die insbesondere einen zum Innenraumdurchmesser dI des Druckschlauches (2) komplementären und insbesondere derart geringfügig größeren Außendurchmesser dA aufweist, dass das Stützelement (2) im Innenraum (14) des Druckschlauches (2) diesen aufdehnt.

5. Vorrichtung nach einem der vorhergehenden Ansprüche, **dadurch gekennzeichnet, dass** der Crimpring (26) oder das dergleichen Fixierelement wenigstens einen Fixierfortsatz (28) aufweist, der im angeordneten Zustand auf der Außenseite (32) des Druckschlauches (2) in Richtung des Innenraums (14) des Druckschlauches (2) vorsteht.

6. Vorrichtung nach einem der vorhergehenden Ansprüche, **dadurch gekennzeichnet, dass** das Anschlagelement (24) wenigstens ein Befestigungsmittel (34) aufweist, über das es mit dem Griffelement (4) und/oder mit dem Halteelement (20) in einen insbesondere lösbaren Arretierungs- oder Stützeingriff bringbar ist.

7. Vorrichtung nach einem der vorhergehenden Ansprüche, **dadurch gekennzeichnet, dass** am freien distalen Ende (3) des Druckschlauches (2) und/oder des Stützelementes (12) ein Einführelement (36) derart angeordnet oder ausgebildet ist, dass es das Einführen des Druckschlauches (2) zusammen mit dem Stützelement (12) in einen Innenraum (38) des O-Rings (18) oder des dergleichen Hüllelementes unterstützt.

## Claims

1. Connector of a surgical instrument (30) or handle for a surgical instrument (30), with a grip element (4) for holding the surgical instrument (30) and at least one pressure hose (2) which is connected to the grip element (4) or the connector via a device (1), by means of which the pressure hose (2) is tightly connected to the grip element (4) or connector of the surgical instrument (30), comprising at least one sealing element (8) which in mounted state is arranged tightly between the grip element (4) and the pressure hose (2),
with a support clement (12) which is formed as a support sleeve (22) and is arranged in the connecting region (10) in the interior (14) of the pressure hose (2) such that the sealing element (8) is clamped between the support element (12) and the pressure hose (2) on the one side and the grip element (4) on the other side,
wherein at least one holding element (20) is arranged on the pressure hose (2) and has a crimping ring (26) which is pressed stationarily onto the outside (32) of the pressure hose (2) over the supporting sleeve,
wherein the holding element (20) is actively connected to a stop element (24) on the grip element (4) for fixing the pressure hose (2) in the axial direction (R_{A}) of the pressure hose (2),
wherein the crimping ring (26) is actively connected to the stop element in the connecting region (10).

2. Device according to claim 1, **characterised in that** the sealing element (8) comprises at least one O-ring (18) or similar cladding element surrounding the pressure hose (2) and supporting element (12) in mounted state.

3. Device according to any of the preceding claims, **characterised in that** the sealing element (8) comprises at least two O-rings (18) or similar cladding elements surrounding the pressure hose (2) and supporting element (12), and decoupled from each other via at least one spacer element (16).

4. Device according to any of the preceding claims, **characterised in that** the support element (12) has a supporting sleeve (22) which in particular has an outer diameter dA which is complementary to and in particular slightly larger than the inner diameter dI of the pressure hose (2), and that the supporting element (2) in the interior (14) of the pressure hose (2) stretches this.

5. Device according to any of the preceding claims, **characterised in that** the crimping ring (26) or similar fixing clement has at least one fixing extension (38) which, in the state arranged on the outside (32) of the pressure hose (2), protrudes in the direction of the interior (14) of the pressure hose (2).

6. Device according to any of the preceding claims, **characterised in that** the stop element (24) has at least one fixing means (34) via which it can be brought into an in particular releasable locking or supporting engagement with the grip element (4) and/or the holding element (20).

7. Device according to any of the preceding claims, **characterised in that** an insertion element (36) is arranged or formed on the free distal end (3) of the pressure hose (2) and/or the supporting element (12) such that it supports the insertion of the pressure hose (2) together with the supporting element (12) in an interior (38) of the O-ring 18) or similar cladding elment.

## Revendications

1. Raccord d'un instrument chirurgical (30) ou manche pour un instrument chirurgical (30), comprenant un élément de poignée (4), destiné à tenir l'instrument chirurgical (30), et au moins un tuyau flexible de pression (2) qui est raccordé à l'élément de poignée (4) ou au raccord, par l'intermédiaire d'un dispositif (1) à l'aide duquel le tuyau de pression (2) est raccordé de façon étanche à l'élément de poignée (4) ou au raccord de l'instrument chirurgical (30), comportant au moins un élément d'étanchéité (8) qui, à l'état de montage, est disposé de manière à établir l'étanchéité entre l'élément de poignée (4) et le tuyau de pression (2),
comprenant un élément de support (12) qui est réalisé sous forme de manchon de support (22) et est disposé, dans la zone de raccordement (10), à l'intérieur (14) du tuyau de pression (2), de manière à ce que l'élément d'étanchéité (8) soit serré entre l'élément de support (12) et le tuyau de pression (2), d'une part, et l'élément de poignée (4), d'autre part,
au moins un élément de maintien (20) étant disposé sur le tuyau de pression (2), lequel élément présente une bague de sertissage (26) qui est pressée de manière fixe sur la face externe (32) du tuyau de pression (2), par-dessus le manchon de support,
ledit élément de maintien (20) étant en liaison fonctionnelle avec un élément de butée (24) prévu sur l'élément de poignée (4), en vue de fixer le tuyau de pression (2) dans le sens axial (R_{A}) du tuyau de pression (2),
la bague de sertissage (26) étant en liaison fonctionnelle avec l'élément de butée, dans la zone de raccordement (10).

2. Dispositif selon la revendication 1, **caractérisé en ce que** l'élément d'étanchéité (8) présente au moins un joint torique (18) ou un élément d'enveloppe analogue entourant le tuyau de pression (2) et l'élément de support (12) à-l'état monté.

3. Dispositif selon l'une des revendications précédentes, **caractérisé en ce que** l'élément d'étanchéité (8) présente au moins deux joints toriques (18) ou des éléments d'enveloppe analogues qui entourent le tuyau de pression (2) et l'élément de support (12) et qui sont découplés l'un de l'autre par au moins un élément d'espacement (16).

4. Dispositif selon l'une des revendications précédentes, **caractérisé en ce que** l'élément de support (12) présente un manchon de support (22) ayant notamment un diamètre extérieur dA qui est complémentaire du diamètre intérieur dI du tuyau de pression (2) et qui est en particulier supérieur à celui-ci dans une mesure tellement faible que l'élément de support (12) se trouvant à l'intérieur (14) du tuyau de pression (2) dilate ce dernier.

5. Dispositif selon l'une des revendications précédentes, **caractérisé en ce que** la bague de sertissage (26) ou l'élément de blocage analogue présente au moins une saillie de blocage (28) qui, à l'état installé sur la face externe (32) du tuyau de pression (2), avance en direction de l'intérieur (14) du tuyau de pression (2).

6. Dispositif selon l'une des revendications précédentes, **caractérisé en ce que** l'élément de butée (24) présente au moins un moyen de fixation (34) par l'intermédiaire duquel il peut être amené en prise de verrouillage ou d'appui, notamment libérable, avec l'élément de poignée (4) et/ou avec l'élément de maintien (20).

7. Dispositif selon l'une des revendications précédentes, **caractérisé en ce que**, à l'extrémité distale (3) libre du tuyau de pression (2) et/ou de l'élément de support (12), un élément d'insertion (36) est disposé ou réalisé de manière telle qu'il favorise l'insertion du tuyau de pression (2), conjointement avec l'élément de support (12), dans un espace intérieur (38) du joint torique (18) ou de l'élément d'enveloppe analogue.
